# EUROPÄISCHE PATENTSCHRIFT

(11) **EP 4 028 720 B1**
(45) Veröffentlichungstag und Bekanntmachung des Hinweises auf die Patenterteilung: **24.07.2024**
(21) Anmeldenummer: 19779377.1
(22) Anmeldetag: 12.09.2019
(51) Int. Cl.: G01B 11/16, A61B 34/20

(54) **VERFAHREN UND VORRICHTUNG ZUR BESTIMMUNG DER FORM EINES LICHTWELLENLEITERS SOWIE VORRICHTUNG ZUR ERZEUGUNG VON TRAININGSDATEN FÜR EIN NEURONALES NETZ**
METHOD AND DEVICE FOR DETERMINING THE SHAPE OF AN OPTICAL WAVEGUIDE, AND DEVICE FOR PRODUCING TRAINING DATA FOR A NEURAL NETWORK
PROCÉDÉ ET DISPOSITIF POUR DÉTERMINER LA FORME D'UN GUIDE D'ONDE OPTIQUE ET DISPOSITIF POUR PRODUIRE DES DONNÉES D'APPRENTISSAGE POUR UN RÉSEAU NEURONAL

(43) Veröffentlichungstag der Anmeldung: 20.07.2022
(73) Patentinhaber: Fraunhofer-Gesellschaft zur Förderung der angewandten Forschung e.V., 80686 München (DE)
(72) Erfinder: SCHADE, Wolfgang, 38644 Goslar (DE)
(74) Vertreter: Friese Goeden Patentanwälte PartGmbB
(86) Internationale Anmeldenummer: PCT/EP2019/074389
(87) Internationale Veröffentlichungsnummer: WO 2021/047779

(56) Entgegenhaltungen:
- DE-A1- 102014 211 918
- US-A1- 2007 297 714
- LUN TIAN LE TIM ET AL: "Real-Time Surface Shape Sensing for Soft and Flexible Structures Using Fiber Bragg Gratings", IEEE ROBOTICS AND AUTOMATION LETTERS, IEEE, vol. 4, no. 2, April 2019 (2019-04-01), pages 1454 - 1461, XP011711122, DOI: 10.1109/LRA.2019.2893036
- ZENG N ET AL: "Enhancement of the measurement range of FBG sensors in a WDM network: a self-organizing network solution", SENSORS AND ACTUATORS A: PHYSICAL, ELSEVIER BV, NL, vol. 118, no. 2, 28 February 2005 (2005-02-28), pages 233 - 237, XP004733549, ISSN: 0924-4247, DOI: 10.1016/J.SNA.2004.08.010
- I M VAN MEERBEEK ET AL: "Soft optoelectronic sensory foams with proprioception", 28 November 2018 (2018-11-28), XP055699638, Retrieved from the Internet <URL:https://robotics.sciencemag.org/content/robotics/3/24/eaau2489.full.pdf> [retrieved on 20200529]
- HANRONG ZHENG ET AL: "Artificial neural network for the reduction of birefringence-induced errors in fiber shape sensors based on cladding waveguides gratings", OPTICS LETTERS, vol. 45, no. 7, 18 February 2020 (2020-02-18), US, pages 1726, XP055699480, ISSN: 0146-9592, DOI: 10.1364/OL.386218

## Beschreibung

Die Erfindung betrifft ein Verfahren und eine Vorrichtung zur Bestimmung der Form eines Lichtwellenleiters mit einer Vielzahl von Faser-Bragg-Gittern, enthaltend die folgenden Schritten: Einkoppeln von Licht einer Lichtquelle in den Lichtwellenleiter, Auskoppeln des reflektierten Lichts aus dem Lichtwellenleiter, Bestimmen des Spektrums des reflektierten Lichts durch Messen der Intensität gegen die Wellenlänge. Verfahren und Vorrichtungen dieser Art können dazu verwendet werden, die Form eines mit zumindest einem Lichtwellenleiter versehenen Gegenstandes zu erfassen.

Aus der DE 10 2016 214 887 A1 ist eine Vorrichtung und ein Verfahren der eingangs genannten Art bekannt. Dieser bekannte faseroptische Sensor weist einen Lichtwellenleiter auf, in welchen Faser-Bragg-Gitter eingebracht sind. die Bragg-Gitter sind radial beabstandet, d.h. in unterschiedlichem Abstand und/oder unterschiedlicher Richtung zur Symmetrieachse des Querschnittes des Lichtwellenleiters angeordnet. Bei Verformung des Lichtwellenleiters ändert sich durch Dehnung auf der Außenseite der Krümmung bzw. Stauchung auf der Innenseite der Krümmung die Gitterkonstante der Bragg-Gitter. Darüber hinaus verschiebt sich das Modenfeld des innerhalb des Lichtwellenleiters propagierenden Lichtes. Hierdurch ändert sich die Reflektionswellenlänge und/oder die Intensität reflektierten Lichtes. Aus diesen Änderungen des Spektrums kann die Krümmung am Ort der Bragg-Gitter bestimmt werden.

Diese bekannte Vorrichtung weist den Nachteil auf, dass eine Krümmung in einem Längsabschnitt, in welchem kein Bragg-Gitter eingebracht ist, unter Umständen nicht nachweisbar ist. Insbesondere die Form von Lichtwellenleitern großer Länge kann dadurch unter Umständen nur unvollständig rekonstruiert werden.

Der Artikel "Real-Time Surface Shape Sensing for Soft and Flexible Structures Using Fiber Bragg Gratings" von Lun Tian Le Tim et al. lehrt ein Verfahren zur Bestimmung der Form eines Lichtwellenleiters, bei dem gemessene Bragg-Wellenlängen zur Formbestimmung einem vorab trainierten neuronalen Netz zugeführt werden.

Ausgehend vom Stand der Technik liegt der Erfindung somit die Aufgabe zugrunde, eine Vorrichtung und ein Verfahren bereitzustellen, welches eine größere Genauigkeit bei der Formbestimmung eines Lichtwellenleiters und eines damit verbundenen Gegenstandes ermöglicht.

Die Aufgabe wird erfindungsgemäß durch ein Verfahren gemäß Anspruch 1, eine Vorrichtung nach Anspruch 9 und eine Vorrichtung nach Anspruch 10 gelöst. Vorteilhafte Weiterbildungen der Erfindung finden sich in den abhängigen Ansprüchen. Erfindungsgemäß wird ein Verfahren zur Bestimmung der Form eines Lichtwellenleiters gemäß Anspruch 1 vorgeschlagen. Der Lichtwellenleiter enthält in an sich bekannter Weise einen Kern und einen den Kern umgebenden Mantel mit unterschiedlicher Brechzahl, so dass Licht, welches in den Kern eingekoppelt ist, an der Grenzfläche zwischen Kern und Mantel totalreflektiert wird und dadurch innerhalb des Kernes propagiert. In einigen Ausführungsformen der Erfindung kann der Kern und der Mantel einen polygonalen oder runden Querschnitt aufweisen und damit die Form einer optischen Faser annehmen. In Abhängigkeit des Durchmessers des Kerns kann der erfindungsgemäße Lichtwellenleiter eine Single Mode Faser oder eine Multi Mode Faser sein bzw. eine solche enthalten. Der erfindungsgemäß vorgeschlagene Lichtwellenleiter kann eine Glasfaser oder eine Polymerfaser sein oder solche Fasern enthalten. In anderen Ausführungsformen der Erfindung kann der Mantel ein flächiges Substrat sein oder ein solches enthalten, wobei zumindest ein Kern innerhalb des Substrates angeordnet ist, beispielsweise durch Einschreiben mit einem Kurzpulslaser.

Erfindungsgemäß wird weiterhin vorgeschlagen, dass der Lichtwellenleiter mit einer Mehrzahl an Faser-Bragg-Gittern versehen ist. Die Bragg-Gitter enthalten jeweils eine Mehrzahl von Raumbereichen bzw. Voxeln, welche einen im Vergleich zu Umgebung veränderten Brechungsindex aufweisen. Die Bragg-Gitter können beispielsweise durch Einschreiben mit einem Kurzpulslaser erzeugt werden. Die Bragg-Gitter können im Inneren des Kerns und/oder im Grenzbereich zwischen Kern und Mantel oder innerhalb des Mantels angeordnet sein, bevorzugt im Evaneszenzbereich des Kerns. Die Anwesenheit von Bragg-Gittern hat die Wirkung, dass Licht einer vorgebbaren, von der Gitterkonstanten des Bragg-Gitters abhängigen Wellenlänge reflektiert wird. Licht anderer Wellenlänge wird hingegen transmittiert. Das Bragg-Gitter wirkt insoweit als Bandpassfilter.

Ändert sich die Gitterkonstante des Bragg-Gitters, beispielsweise durch Torsion des Lichtwellenleiters durch Dehnung oder Stauchung des Lichtwellenleiters oder durch Krümmung, so ändert sich auch die Wellenlänge des vom Bragg-Gitter reflektierten Lichtes. Durch Laufzeitmessung und/oder Wellenlängen-Multiplex kann der Ort des jeweiligen Bragg-Gitters innerhalb der Faser diskriminiert werden.

Zur Durchführung des erfindungsgemäßen Verfahrens zur Bestimmung der Form des Lichtwellenleiters wird Licht einer Lichtquelle in den Lichtwellenleiter eingekoppelt. Die Lichtquelle kann in einigen Ausführungsformen ausgewählt sein aus einem Halbleiterlaser und/oder einer Leuchtdiode und/oder einer Superlumineszenzdiode. Die Lichtquelle kann in einigen Ausführungsformen der Erfindung breitbandiges Licht emittieren, so dass unterschiedliche Teilspektren des eingekoppelten Lichtes an unterschiedlichen Bragg-Gittern innerhalb der Faser reflektiert werden.

Das solchermaßen in den Lichtwellenleiter eingekoppelte Licht wird zumindest teilweise innerhalb des Lichtwellenleiters reflektiert. Die Reflektion kann einerseits an den im Lichtwellenleiter vorhandenen Bragg-Gittern erfolgen. In anderen Ausführungsformen der Erfindung kann das der Lichtquelle entgegengesetzte, frei Ende des Lichtwellenleiters verspiegelt sein, so dass dort ebenfalls zumindest ein Teil des eingekoppelten Lichtes reflektiert wird.

Das reflektierte Licht aus dem Lichtwellenleiter wird nachfolgend spektral analysiert, d.h. das reflektierte Licht wird einem Spektrometer zugeführt, welches die Lichtintensität gegen die Wellenlänge bestimmt. Hierzu kann das Spektrometer in einigen Ausführungsformen der Erfindung zumindest ein mikrooptisches Bauelement als planaroptisches Filterelement enthalten. Das planaroptische Filterelement kann ausgewählt sein aus einem Arrayed-Waveguide-Grating und/oder einem Mach-Zehnder-Interferometer und/oder einem Delayline-Interferometer und/oder einem Richtkoppler. Hierdurch kann sich eine robuste Bauweise der erfindungsgemäßen Vorrichtung ergeben, welche einen zuverlässigen Einsatz ermöglicht.

Zur Bestimmung der Lichtintensität kann in einigen Ausführungsformen der Erfindung eine Fotodiode oder ein Photodiodenarray oder ein CMOS-Sensor oder ein CCD-Sensor eingesetzt werden. Ein Photodiodenarray oder ein CMOS-Sensor oder ein CCD-Sensor kann als Flächensensor oder als Zeilensensor ausgestaltet sein. Diese Bauelemente lassen sich in einfacher Weise mit dem mikrooptischen Bauelement integrieren.

Erfindungsgemäß wird nun vorgeschlagen, das solchermaßen erfasste Spektrum einem selbst lernenden neuronalen Netz zuzuführen und von diesem die Form des Lichtwellenleiters aus dem gemessenen Spektrum ermitteln zu lassen. Das neuronale Netz weist dabei den Vorteil auf, dass dieses nicht nur die Intensität und die Wellenlänge des an den Bragg-Gittern reflektierten Lichtes auswertet, sondern darüber hinaus auch die Intensität und Art des Untergrundsignals als weiteres Kriterium zur Bestimmung der Form des Lichtwellenleiters heranzieht. Voraussetzung hierfür ist, dass das neuronale Netz durch Trainingsdaten konditioniert wurde, d.h. dem selbstlernenden, neuronalen Netz werden bekannte Formen des Lichtwellenleiters und sich daraus ergebende Spektren des reflektierten Lichtes zugeführt. Das selbstlernende neuronale Netz stellt dabei eine künstliche Intelligenz dar, welche sich anhand dieser Trainingsdaten selbst optimiert und so nach und nach in der Lage ist, nicht nur die exakte, vorher angelernte Form aus dem zugeführten Spektrum wiederzuerkennen, sondern auch abweichende Formen aus abweichenden Spektren durch Interpolation zuverlässig zu erkennen. Ein solchermaßen trainiertes neuronales Netz kann somit auch Polarisationseffekte oder Torsion des Lichtwellenleiters erkennen, welche im Falle bekannter statischer Auswertealgorithmen als Artefakte die Formbestimmung des Lichtwellenleiters unmöglich machen. Hierdurch wird die Zuverlässigkeit der Formerkennung erhöht.

In einigen Ausführungsformen der Erfindung können die Trainingsdaten mehr als 600 oder mehr als 900 oder mehr als 4000 oder mehr als 10000 oder mehr als 100000 Datensätze enthalten. Ein Datensatz umfasst dabei jeweils ein gemessenes Spektrum und die zugehörige Form des Lichtwellenleiters. Je größer die Anzahl der Trainingsdaten ist, um so besser passt sich die das selbstlernende, neuronale Netz enthaltende künstliche Intelligenz an die Aufgabe der Formbestimmung an, so dass die erhaltenen Ergebnisse im realen Betrieb entsprechend genauer werden.

In einigen Ausführungsformen der Erfindung können die Trainingsdaten automatisiert mit einer Vorrichtung erstellt werden, welche dazu eingerichtet ist, den Lichtwellenleiter zu verformen, die Form zu erfassen und das zur jeweiligen Form gehörigen Spektrum zu messen. Nach der Erfassung der Form des Lichtwellenleiters und des zugehörigen Spektrums kann die Vorrichtung automatisiert den Wellenleiter in eine andere Form bringen und sodann erneut dessen Form und das zugehörige Spektrum erfassen. Auf diese Weise können in kurzer Zeit große Datenmengen an Trainingsdaten erzeugt werden. Diese Trainingsdaten können neben der dreidimensionalen Krümmung des Lichtwellenleiters auch Torsionsspannungen des Wellenleiters umfassen.

In einigen Ausführungsformen der Erfindung kann das Verformen des Lichtwellenleiters durch einen Roboterarm erfolgen. Ein solcher Roboterarm ist ein multifunktionaler Handhabungsautomat, der aus einer Reihe von starren Gliedern besteht, die miteinander durch Dreh- und/oder Schubgelenke verbunden sind. die Gelenke werden durch gesteuerte Antriebe verstellt. Ein Ende der solchermaßen gebildeten Gliederkette ist die Basis, mit welcher der Roboterarm beispielsweise auf einer Tischplatte oder einer optischen Bank befestigt ist. Das andere Ende ist frei beweglich und mit einem Werkzeug oder Greifer zur Aufnahme eines Längsabschnittes des Lichtwellenleiters bestückt. Somit kann durch Bewegung des Roboterarms der Lichtwellenleiter in eine definierte Form gebracht werden. Der Roboterarm kann so programmiert werden, dass dieser den Lichtwellenleiter systematisch in ein, zwei oder drei Raumrichtungen und/oder ein, zwei oder drei Drehachsen bewegt, so dass ein vollständiger Satz an Trainingsdaten für einen vorgebbaren Anwendungsfall erhalten werden kann.

In einigen Ausführungsformen der Erfindung kann das Erfassen der Form des Lichtwellenleiters aus der Position des Roboterarms erfolgen. Als Position des Roboterarms können entweder die ausgegebenen Sollwerte oder die vom Roboterarm nach Anfahren der jeweiligen Position zurückgemeldeten IstWerte verwendet werden. Aus der relativen Lage des Endes des Roboterarms und einer Einspannstelle des Lichtwellenleiter sowie dessen Länge und Elastizitätsmodul kann sodann bestimmt werden, welchen Krümmungsverlauf der Lichtwellenleiter im dreidimensionalen Raum annimmt.

In einigen Ausführungsformen der Erfindung kann das Erfassen der Form des Lichtwellenleiters durch elektronische Bildverarbeitung aus zumindest einer Aufnahme des Lichtwellenleiters erfolgen. In wiederum einer anderen Ausführungsform der Erfindung kann die Form des Lichtwellenleiters sowohl aus der Position des Roboterarms als auch aus zumindest einer fotografischen Aufnahme des Lichtwellenleiters erfolgen. Hierdurch können beide Messungen gegeneinander plausibilisiert werden und die Genauigkeit der Formbestimmung bei der Erstellung der Trainingsdaten erhöht sein.

Ein erfindungsgemäßer Lichtwellenleiter kann mit einem beweglichen Bauelement versehen sein und auf diese Weise dessen Form und Lage im Raum erfassen. Ein erfindungsgemäßes Bauelement kann beispielsweise ausgewählt sein aus einem Katheter oder einem Endoskop oder einer Biopsienadel oder aus einem aerodynamischen Profil, beispielsweise einem Segel oder einer Segellatte.

Nachfolgend soll die Erfindung anhand von Figuren ohne Beschränkung der beanspruchten Erfindung näher erläutert werden. Dabei zeigt
Fig. 1 das der Erfindung zugrundeliegende Messprinzip.
Fig. 2 zeigt ein Blockschaltbild einer erfindungsgemäßen Vorrichtung zur Bestimmung der Form eines Lichtwellenleiters.
Fig. 3 zeigt ein Blockschaltbild einer erfindungsgemäßen Vorrichtung zur Erzeugung von Trainingsdaten.
Fig. 4 zeigt unterschiedliche Formen eines Lichtwellenleiters.
Fig. 5 zeigt die bei Formänderung erhaltenen Spektren.
Fig. 6 zeigt die Güte der Formrekonstruktion in Abhängigkeit der Menge der Trainingsdaten.

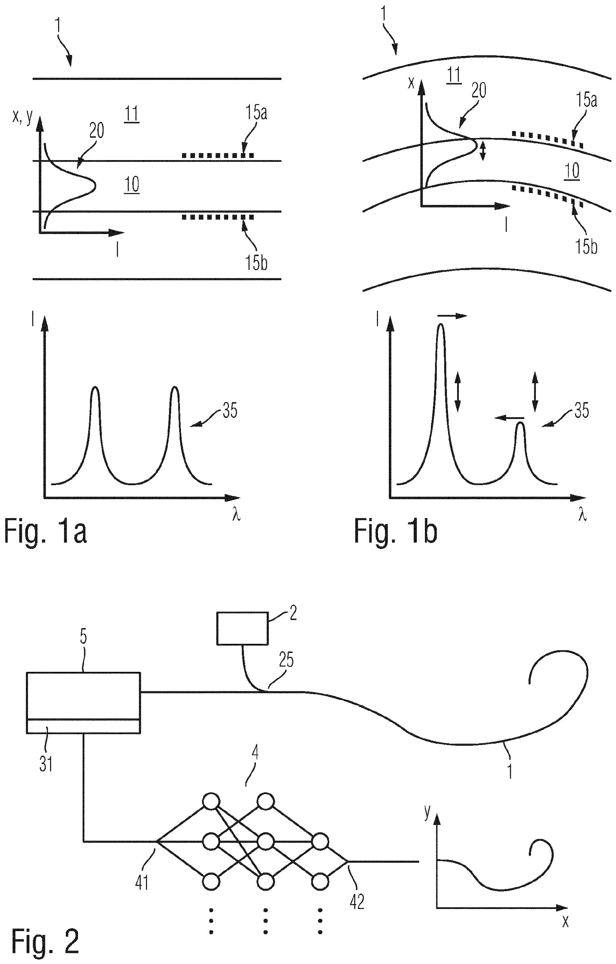

Anhand der Fig. 1 wird das der Erfindung zugrundeliegende Messprinzip erläutert. Dabei zeigt Fig. 1A einen geraden Lichtwellenleiter und Fig. 1B einen gekrümmten Lichtwellenleiter.

In jedem Fall weist der Lichtwellenleiter 1 einen Kern 10 und einen den Kern umgebenden Mantel 11 auf. Beispielsweise kann der Mantel 11 den Kern 10 konzentrisch umgeben. Kern und Mantel können aus Glas oder Polymer gefertigt sein. Zwischen dem Kern 10 und dem Mantel 11 befindet eine Grenzfläche, an welcher im Kern 10 propagierendes Licht 20 totalreflektiert wird. Hierdurch kann das Licht im Kern 10 geführt werden.

Weiterhin befindet sich im Lichtwellenleiter zumindest ein Bragg-Gitter 15. Im dargestellten Ausführungsbeispiel sind zwei Bragg-Gitter 15A und 15B im selben Längsabschnitt des Lichtwellenleiters 1 vorhanden. Die Bragg-Gitter 15 bestehen jeweils aus einer Mehrzahl von Raumbereichen bzw. Voxeln, welche einen anderen Brechungsindex aufweisen als das umgebende Material. Der Abstand benachbarter Voxel definiert die Gitterkonstante. Die Wirkung des Bragg-Gitters 15 besteht darin, dass Licht einer vorgebbaren Wellenlänge, welche durch die Gitterkonstante definiert ist, reflektiert wird. Licht einer anderen Wellenlänge kann das Bragg-Gitter 15 im Wesentlichen ungestört passieren.

Die Bragg-Gitter 15 können im Kern angeordnet sein. Im dargestellten Ausführungsbeispiel sind die Bragg-Gitter 15 im Evaneszenzbereich des Mantels angeordnet. Der Evaneszenzbereich ist dadurch definiert, dass ein Teil der Intensität des im Kern propagierendes Lichtes in diesen eindringt, obgleich die Totalreflektion nominell an der Grenzfläche stattfindet.

Fig. 1A zeigt weiterhin ein beispielhaftes Modenfeld des Lichtes 20, welches im Kern propagiert. Dargestellt ist die Intensität gegen die Ortskoordinate des Querschnittes des Lichtwellenleiters 1. Dabei ist ersichtlich, dass ein Teil der Intensität in den Kern 11 eintritt und so mit den im Evaneszenzbereich angeordneten Bragg-Gittern 15 wechselwirken kann.

Fig. 1B zeigt, dass bei einer Krümmung des Lichtwellenleiters 1 das äußere Bragg-Gitter 15A gedehnt und das innenliegende Bragg-Gitter 15B gestaucht wird. Dies kann zu einer Änderung der Gitterkonstanten führen, welche über eine Verschiebung des Intensitätsmaximums des reflektierten Lichtes erkannt werden kann. Darüber hinaus verschiebt sich das Modenfeld 20 relativ zum Kern 10. Dies führt zu einem Anwachsen der Intensität reflektierten Lichtes im Bragg-Gitter 15A auf der Außenseite der Krümmung und zu einer Abnahme der Intensität auf der Innenseite.

Die Fig. 1A und 1B zeigen jeweils ein Spektrum 35, welches an einem geraden und einem gekrümmten Wellenleiter gemessen werden kann. Neben der Verschiebung der Lage der Reflektionsmaxima und deren Intensität kann durch die Verschiebung des Modenfeldes 20 des propagierenden Lichtes auch das zwischen den Intensitätsmaxima liegende Untergrundfeld verändert werden.

Anhand der Fig. 2 wird eine Vorrichtung zur Bestimmung der Form eines Lichtwellenleiters näher erläutert. Die Vorrichtung gemäß Fig. 2 zeigt einen Lichtwellenleiter 1. Dieser kann an einem mechanischen Gegenstand befestigt sein, dessen Formänderung erfasst werden soll. Beispielsweise kann dieser Gegenstand eine Biopsienadel und/oder ein Endoskop und/oder einen Katheter und/oder ein aerodynamisches Profil enthalten oder daraus bestehen. Ein aerodynamisches Profil kann beispielsweise ein Segel sein oder ein Teil eines Segels, beispielsweise eine Segellatte. Das Segel kann aus einem Folienmaterial laminiert oder aus einem textilen Material gewebt sein. Der Lichtwellenleiter 1 kann dabei aufgeklebt oder eingewebt oder einlaminiert sein. Optional kann ein solches Segel durch Segellatten stabilisiert werden. Auch die Segellatten, welche beispielsweise aus faserverstärktem Kunststoff gefertigt sind, können mit einem Lichtwellenleiter 1 ausgestattet sein.

Die Vorrichtung gemäß Fig. 2 enthält weiterhin eine Lichtquelle 2, welche beispielsweise eine Superlumineszenzdiode oder eine Leuchtdiode sein kann. Die Lichtquelle 2 kann breitbandige Strahlung aussenden, welche die durch die Gitterkonstante der Bragg-Gitter 15 definierten Bragg-Wellenlängen abdeckt.

Das Licht der Lichtquelle 2 wird über einen Koppler 25 in den Lichtwellenleiter 1 eingekoppelt und propagiert in dessen Kern 10, wie vorstehend beschrieben. Bei Wechselwirkung mit den Bragg-Gittern 15 wird ein Teil des propagierenden Lichtes in die Einfallsrichtung zurückreflektiert. Darüber hinaus kann das Licht absorbiert werden, beispielsweise durch Gitterfehler oder Störstellen im Gefüge des Lichtwellenleiters 1. Schließlich kann ein Teil der Intensität durch evaneszente Kopplung in den Mantel 11 eindringen und/oder den Lichtwellenleiter 1 verlassen.

Das solchermaßen modifizierte, reflektierte Licht gelangt in ein Spektrometer 3, welches beispielsweise als integrierte mikrooptische Komponente ausgeführt sein kann. Beispielhaft kann eine solche mikrooptische Komponente als AWG ausgeführt sein. Das Spektrometer 3 bildet Licht unterschiedlicher Wellenlänge an unterschiedlichen Orten ab. Dort kann das Spektrum durch auf einen ortsauflösenden Detektor 31 erfasst werden.

Der ortsauflösende Detektor 31 kann beispielsweise ein Photodiodenarray, einen CCD-Sensor oder einen CMOS-Sensor enthalten. Die vorgenannten Sensoren können beispielsweise als Zeilen- oder als Flächensensor ausgeführt sein.

Das elektrische Signal des ortsauflösenden Detektors 31 kann optional einem A/D-Wandler zugeführt und nachfolgend als digitales Signal weiterverarbeitet werden. Dieses das Spektrum 35 repräsentierende Signal wird einem neuronalen Netz 4 bzw. einer künstlichen Intelligenz mit einem Eingang 41 und einem Ausgang 42 zugeführt.

Das neuronale Netz 4 enthält eine Mehrzahl von Knoten bzw. Neuronen. Diese können in einer Eingangsebene und einer Ausgangsebene und zumindest einer Mittelebene organisiert sein. Das neuronale Netz 4 ist ein selbstlernendes neuronales Netz bzw. eine künstliche Intelligenz, d.h. die Verbindungen zwischen einzelnen Neuronen ändern sich bei Betrieb des neuronalen Netzes 4, so dass die Qualität der Erkennung mit zunehmender Anzahl von Trainingsdaten bzw. längerem Betrieb ansteigt. Am Ausgang 42 des neuronalen Netzes 4 stehen sodann Daten zur Verfügung, welche die zwei- oder dreidimensionale Form des Lichtwellenleiters 1 repräsentieren. Erfindungsgemäß wurde erkannt, dass durch Verwendung des neuronales Netzes die Bestimmung der Form des Lichtwellenleiters 1 aus dem Spektrum 35 mit größerer Genauigkeit erfolgen kann. Dies insbesondere deshalb, weil auch das sich bei Krümmung ändernde Untergrundsignal zur Plausibilisierung bzw. Bestimmung der Form herangezogen wird und anders als bei bekannten Auswertungen die Form nicht nur aus der Position und/oder der Intensität der Reflektionsmaxima bestimmt wird. Damit kann die Form eines mit dem Lichtwellenleiter 1 verbundenen Gegenstandes mit größerer Genauigkeit bestimmt werden.

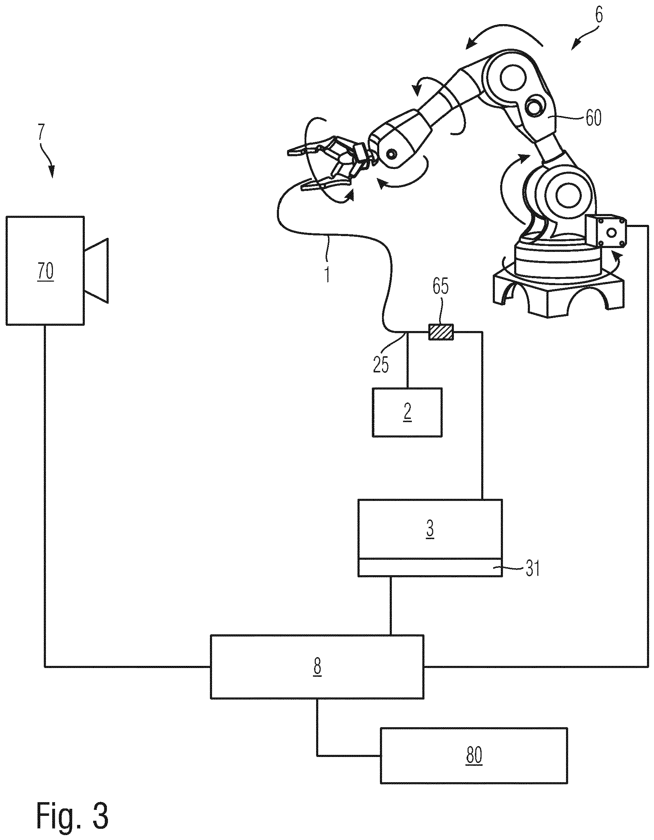

Fig. 3 zeigt eine Vorrichtung zur Erzeugung von Trainingsdaten für ein neuronales Netz 4. Da die Qualität der Formbestimmung des Lichtwellenleiters 1 mit der Qualität der Trainingsdaten korreliert ist, ist es vorteilhaft, in endlicher Zeit eine möglichst große Anzahl an Trainingsdaten zu erzeugen. Die hierfür einsetzbare Vorrichtung wird anhand der Fig. 3 näher erläutert. Fig. 3 zeigt einen Lichtwellenleiter 1, welchem über einen Koppler 21 Licht über eine Lichtquelle 2 zuführbar ist, wie vorstehend beschrieben. Das vom Lichtwellenleiter 1 reflektierte Licht wird einem Spektrometer 3 und einem ortsauflösenden Detektor 31 zugeführt, wie vorstehend beschrieben. Optional kann ein A/D-Wandler vorhanden sein, welcher die Daten des ortsauflösenden Detektors 31 digitalisiert.

Der Lichtwellenleiter 1 ist mit einem Ende oder einem Längsabschnitt in einer Einspannstelle 65 gehalten. Ein gegenüberliegendes Ende oder ein beanstandeter Längsabschnitt ist in einer Einrichtung 6 zur Verformung des Lichtwellenleiters aufgenommen. Durch die Einrichtung 6 zur Verformung des Lichtwellenleiters kann der Lichtwellenleiter in eine gekrümmte, vom geradlinigen Verlauf abweichende Form gebracht werden. Hierzu kann die Einrichtung 6 zur Verformung beispielsweise einen Roboterarm 6 aufweisen. Der Roboterarm ist um mehrere Achsen drehbar und/oder schwenkbar ausgeführt, so dass der Lichtwellenleiter 1 in ein, zwei oder drei Raumrichtungen und/oder in ein, zwei oder drei Drehrichtungen verformt werden kann.

Die Form des Lichtwellenleiters 1 kann sodann mit einer Einrichtung 7 zur Erfassung der Form bestimmt werden. Die Einrichtung 7 zur Erfassung der Form kann beispielsweise eine digitale Kamera 70 enthalten, welche mittels eines CCD- oder CMOS-Sensors und eines Objektivs einen digitalen Datenstrom erzeugt, welcher die Form des Lichtwellenleiters 1 repräsentiert.

Alternativ oder zusätzlich kann die Einrichtung 7 zur Erfassung der Form in der Einrichtung 6 zur Verformung des Lichtwellenleiters 1 integriert sein, bzw. die Form des Lichtwellenleiters 1 kann aus der Position der Einrichtung 6 zur Verformung rechnerisch bestimmt werden. Sodann wird die erfasste Form des Lichtwellenleiters zusammen mit dem gemessenen Spektrum 35 in einem Speicher 80 abgelegt. Danach wird der Lichtwellenleiter 1 in eine andere Form gebracht, die Form wird erfasst und das Spektrum reflektierten Lichtes gemessen. Auch diese Daten werden im Speicher 80 abgelegt. Sodann wird dieses Vorgehen zyklisch wiederholt. Ein Mikroprozessor bzw. ein Mikrocontroller 8 übernimmt die Datenerfassung sowie die Ansteuerung der Einrichtung 7 zur Erfassung der Form sowie der Einrichtung 6 zur Verformung des Lichtwellenleiters 1, so dass verschiedene Formen des Lichtwellenleiters systematisch durchfahren werden können bzw. eine Vielzahl statistisch ausgewählter Formen erfasst werden kann. Solchermaßen können in einigen Ausführungsformen der Erfindung mehr als 600 oder mehr als 900 oder mehr als 4000 oder mehr als 10000 oder mehr als 100000 Datensätze erzeugt werden, wobei jeder Datensatz zumindest eine Form des Lichtwellenleiters 1 und ein gemessenes Spektrum 35 erhält. Anhand dieser Daten kann schließlich das neuronale Netz 4 trainiert werden, so dass dieses anders als eine Umsetzungstabelle nicht nur einem konkret gemessenen Spektrum eine Form zuweist, sondern auch dann zu einem sinnvollen Ergebnis gelangt, wenn im realen Betrieb eine Form vorliegt, welche in den Trainingsdaten nicht identisch enthalten ist bzw. ein Spektrum erfasst wird, welches nicht identisch in den Trainingsdaten enthalten ist. Das erfindungsgemäß eingesetzte neuronale Netz 4 ermöglicht durch Interpolation bzw. Ähnlichkeitsbetrachtungen auch in diesem Fall die Rekonstruktion der Form.

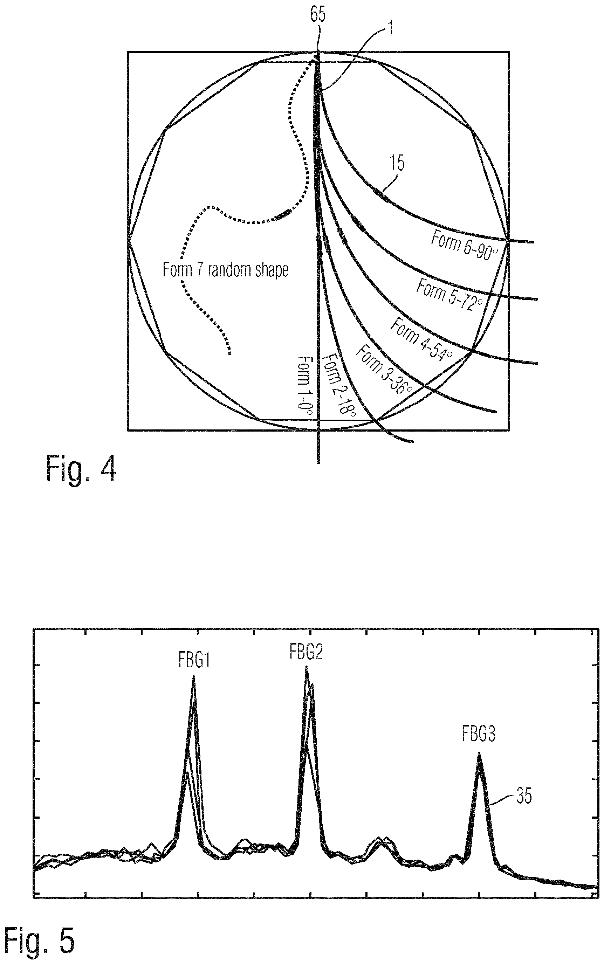

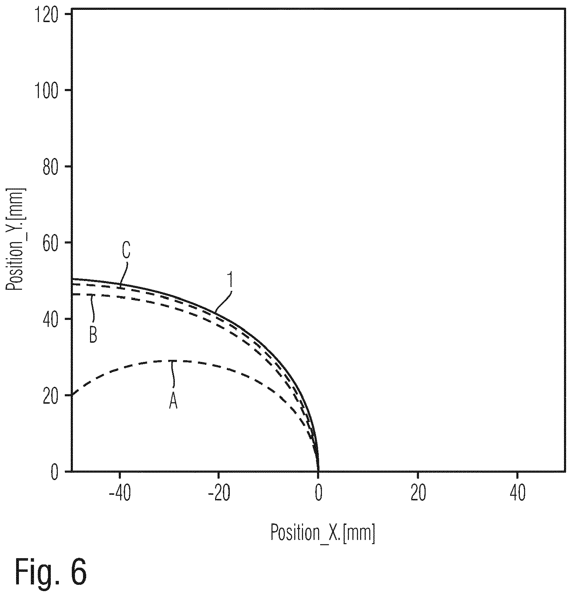

Nachfolgend soll die Erfindung anhand eines Ausführungsbeispiels näher erläutert werden. Dabei zeigt Fig. 4 unterschiedliche Formen eines Lichtwellenleiters, Fig. 5 zeigt die jeweils zugeordneten Spektren 35 und Fig. 6 zeigt die Formrekonstruktion in Abhängigkeit der Güte der Trainingsdaten.

Wie anhand von Fig. 4 erläutert wird, wird beispielhaft ein Lichtwellenleiter 1 betrachtet, welcher eine Einspannstelle 65 aufweist. Das der Einspannstelle 65 gegenüberliegende freie Ende wird einmal um 0°, sodann um 18°, 36°, 54°, 72° und 90° innerhalb einer Ebene verschwenkt. Hierdurch ergibt sich eine Krümmung des Lichtwellenleiters 1 in einer Dimension.

Wie Fig. 5 zeigt, ändert sich für jeden Biegewinkel bzw. jeden Krümmungsradius das gemessene Spektrum von drei Faser-Bragg-Gittern, welche allesamt in einem Längsabschnitt des Lichtwellenleiters 1 angeordnet sind. So variiert einerseits die gemessene Intensität des reflektierten Lichtes in den drei durch die Gitterkonstante festgelegten Reflektionswellenlängen. Darüber hinaus ändert sich jedoch auch der zwischen den Intensitätsmaxima liegende Untergrund.

Diese Daten werden als Trainingsdaten erfasst und zum Anlernen eines neuronalen Netzes verwendet, wie anhand von Fig. 2 beschrieben. In Fig. 6 werden rekonstruierte Formen gezeigt, welche mit einem Aufbau gemäß Fig. 2 erhalten wurden. Dabei ist die Position des Lichtwellenleiters in der in Fig. 4 gezeigten Ebene auf der Ordinate bzw. der Abszisse aufgezeichnet. Dargestellt ist der tatsächliche Verlauf des Lichtwellenleiters 1. Weiterhin dargestellt sind Messkurven A, B und C, welche die vom neuronalen Netz 4 rekonstruierte Form des Lichtwellenleiters 1 zeigen.

Aus Fig. 6 ist ersichtlich, dass die Rekonstruktion gemäß Kurve C bis auf einen geringen Messfehler dem tatsächlichen Verlauf des Lichtwellenleiters folgt. Die Abweichungen der gemessenen von der tatsächlichen Form sind in Fig. B geringfügig größer. Fig. A zeigt eine erhebliche Abweichung, welche in der Praxis unbrauchbar ist.

Die Kurven A, B und C wurden sämtlich mit der identischen, in Fig. 2 gezeigten Vorrichtung erhalten, der Unterschied besteht jeweils in der Menge der Trainingsdaten, welche zum Anlernen des neuronalen Netzes 4 verwendet wurden. Kurve A zeigt ein neuronales Netz, welchem lediglich ein einziger Datensatz zugeführt wurde, d.h. es wurde nur ein einziges Spektrum verwendet, welches bei genau einer einzigen Krümmung erfasst wurde.

Kurve B zeigt das Ergebnis eines neuronalen Netzes, welche mit 600 Datensätzen trainiert wurde. Kurve C wurde mit einem neuronalen Netz erhalten, welches mit 900 Datensätzen trainiert wurde. Fig. 6 zeigt, dass bereits 600 Datensätze ausreichen, um das neuronale Netz so zu trainieren, dass dieses geringe Genauigkeitsanforderungen erfüllt, d.h. einen Fehler von wenigen Millimetern erzeugt. Bei der Verwendung von 900 Datensätzen ist die Rekonstruktion so genau, dass der Fehler unterhalb eines Millimeters liegt. Diese vergleichsweise geringe Anzahl von Trainingsdaten kann unter Verwendung der erfindungsgemäß vorgestellten Vorrichtung auch in kurzer Zeit erhalten werden. In gleicher Art wie vorliegend bei einem einfachen zweidimensionalen Fall gezeigt, können mit der erfindungsgemäßen Vorrichtung und dem erfindungsgemäßen Verfahren auch komplexe Formen im dreidimensionalen Raum rekonstruiert werden.

Selbstverständlich ist die Erfindung nicht auf die dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal die Anwesenheit weiterer Merkmale nicht ausschließt. Sofern die Ansprüche und die vorstehende Beschreibung "erste" und "zweite" Ausführungsformen definieren, so dient diese Bezeichnung der Unterscheidung zweier gleichartiger Ausführungsformen, ohne eine Rangfolge festzulegen.

## Patentansprüche

1. Verfahren zur Bestimmung der Form eines Lichtwellenleiters (1) mit einer Mehrzahl von Faser-Bragg-Gittern (15), enthaltend die folgenden Schritte: Einkoppeln von Licht (20) einer Lichtquelle (2) in den Lichtwellenleiter (1),
Auskoppeln des reflektierten Lichts aus dem Lichtwellenleiter (1),
Bestimmen eines Spektrums (35) des reflektierten Lichts durch Messen der Intensität (I) gegen die Wellenlänge (λ),
**dadurch gekennzeichnet, dass**
das Spektrum (35) einem selbstlernenden, neuronalen Netz (4) zugeführt wird, welches zuvor mit Trainingsdaten angelernt wurde, welche aus einer Mehrzahl von Datensätzen bestehen, welche jeweils ein gemessenes Spektrum (35) und die zugehörige Form des Lichtwellenleiters (1) enthalten, wobei mit dem neuronalen Netz (4) aus Intensität und Wellenlänge des an den Bragg-Gittern reflektierten Lichtes und der Intensität und Art des Untergrundes die Form des Lichtwellenleiters (1) ermittelt wird.

2. Verfahren nach Anspruch 1, **dadurch gekennzeichnet, dass** das selbstlernende, neuronalen Netz mit Trainingsdaten angelernt wird, welche aus einer Mehrzahl von Datensätzen bestehen, welche jeweils ein gemessenes Spektrum (35) und die zugehörige Form des Lichtwellenleiters (1) enthalten.

3. Verfahren nach Anspruch 2, **dadurch gekennzeichnet, dass** die Trainingsdaten mehr als 600 oder mehr als 900 oder mehr als 4.000 oder mehr als 10.000 oder mehr als 100.000 Datensätze enthalten.

4. Verfahren nach einem der Ansprüche 2 oder 3, **dadurch gekennzeichnet, dass** die Trainingsdaten automatisiert mit einer Vorrichtung (5) erstellt werden, welche dazu eingerichtet ist, den Lichtwellenleiter (1) zu verformen, die Form zu erfassen und das zur jeweiligen Form gehörige Spektrum (35) zu messen.

5. Verfahren nach Anspruch 4, **dadurch gekennzeichnet, dass** das Verformen des Lichtwellenleiters (1) durch einen Roboterarm (60) erfolgt.

6. Verfahren nach Anspruch 5, **dadurch gekennzeichnet, dass** das Erfassen der Form des Lichtwellenleiters (1) aus der Position des Roboterarms (60) erfolgt

7. Verfahren nach einem der Ansprüche 4 bis 6, **dadurch gekennzeichnet, dass** das Erfassen der Form des Lichtwellenleiters (1) durch elektronische Bildverarbeitung aus zumindest einer Aufnahme des Lichtwellenleiters (1) erfolgt.

8. Verwendung eines Verfahren nach einem der Ansprüche 1 bis 7 zur Bestimmung der Form eines Katheters oder eines Endoskops oder einer Biopsienadel oder eines aerodynamischen Profils.

9. Vorrichtung zur Bestimmung der Form eines Lichtwellenleiters (1) , enthaltend
zumindest einen Lichtwellenleiter (1) mit einer Mehrzahl von Faser-Bragg-Gittern (15),
zumindest eine Lichtquelle (2), welche dazu eingerichtet ist, Licht in den Lichtwellenleiter (1) einzukoppeln,
zumindest ein Spektrometer (3), welches dazu eingerichtet ist, ein Spektrum (35) des im Lichtwellenleiter (1) reflektierten Lichts durch Messen der Intensität (I) gegen die Wellenlänge (λ) des reflektierten Lichts zu bestimmen,
**dadurch gekennzeichnet, dass**
die Vorrichtung weiterhin ein selbstlernendes, neuronales Netz (4) enthält, welches zuvor mit Trainingsdaten angelernt wurde, welche aus einer Mehrzahl von Datensätzen bestehen, welche jeweils ein gemessenes Spektrum (35) und die zugehörige Form des Lichtwellenleiters (1) enthalten, und
welches zumindest einen Eingang (41) und zumindest einen Ausgang (42) aufweist, wobei dem Eingang (41) das Spektrum zuführbar ist und am Ausgang (42) die Krümmung und/oder die Torsion repräsentierende Daten bereitstellbar sind, welche mit dem neuronalen Netz aus Intensität und
Wellenlänge des an den Bragg-Gittern reflektierten Lichtes und der Intensität und Art des Untergrundes ermittelbar sind.

10. Vorrichtung zur Erzeugung von Trainingsdaten für ein neuronales Netz (4), enthaltend
zumindest einen Lichtwellenleiter (1) mit einer Mehrzahl von Faser-Bragg-Gittern (15),
zumindest eine Lichtquelle (2), welche dazu eingerichtet ist, Licht (20) in den Lichtwellenleiter (1) einzukoppeln,
zumindest einem Spektrometer (3), welches dazu eingerichtet ist, ein Spektrum (35) des im Lichtwellenleiter (1) reflektierten Lichts durch Messen der Intensität (I) gegen die Wellenlänge (λ) des reflektierten Lichts zu bestimmen,
eine Einrichtung zur Verformung (6) des Lichtwellenleiters (1), welche einen Roboterarm (60) enthält oder daraus besteht, und
eine Einrichtung (7) zur Erfassung der Form des Lichtwellenleiters (1) aus der Position des Roboterarms (60), und
einen Speicher (80), in welchen die Form des Lichtwellenleiters (1) und das Spektrum ablegbar sind.

11. Vorrichtung nach Anspruch 10, **dadurch gekennzeichnet, dass**
die Einrichtung (7) zur Erfassung der Form des Lichtwellenleiters (1) eine Kamera (70) enthält,
welche einen ein Bild des Lichtwellenleiters (1) repräsentierenden Datenstrom erzeugt.

## Claims

1. Method for determining the shape of an optical waveguide (1) having a plurality of fiber Bragg gratings (15), comprising the following steps: coupling light (20) of a light source (2) into the optical waveguide (1),
coupling the reflected light out of the optical waveguide (1), determining a spectrum (35) of the reflected light by measuring the intensity (I) versus the wavelength (λ),
**characterized in that**
the spectrum (35) is supplied to a self-learning neural network (4), which was previously trained with training data consisting of a plurality of data sets each containing a measured spectrum (35) and the associated shape of the optical waveguide (1), the shape of the optical waveguide (1) being determined with the neural network (4) from the intensity and wavelength of the light reflected by the Bragg gratings and the intensity and kind of the underground.

2. Method according to claim 1, **characterized in that** the self-learning neural network is trained with training data consisting of a plurality of data sets each containing a measured spectrum (35) and the associated shape of the optical waveguide (1).

3. Method according to claim 2, **characterized in that** the training data contains more than 600 or more than 900 or more than 4,000 or more than 10,000 or more than 100,000 data sets.

4. Method according to any of claims 2 or 3, **characterized in that** the training data is produced in automated fashion with a device (5), which is designed to deform the optical waveguide (1), to detect the shape, and to measure the spectrum (35) associated with the respective shape.

5. Method according to claim 4, **characterized in that** the optical waveguide (1) is deformed by a robot arm (60).

6. Method according to claim 5, **characterized in that** the shape of the optical waveguide (1) is detected from the position of the robot arm (60).

7. Method according to any of claims 4 to 6, **characterized in that** the shape of the optical waveguide (1) is detected by electronic image processing from at least one image of the optical waveguide (1).

8. Use of a method according to any of claims 1 to 7 for determining the shape of a catheter or an endoscope or a biopsy needle or an aerodynamic profile.

9. Device for determining the shape of an optical waveguide (1), comprising
at least one optical waveguide (1) having a plurality of fiber Bragg gratings (15),
at least one light source (2) designed to couple light into the optical waveguide (1),
at least one spectrometer (3) which is designed to determine a spectrum (35) of the light reflected in the optical waveguide (1) by measuring the intensity (I) versus the wavelength (λ) of the reflected light,
**characterized in that**
the device further comprises a self-learning neural network (4) which was previously trained with training data consisting of a plurality of data sets each containing a measured spectrum (35) and the associated shape of the optical waveguide (1), and which has at least one input (41) and at least one output (42), the spectrum being able to be supplied to the input (41) and data representing the curvature and/or the torsion being able to be provided at the output (42)
which can be determined with the neural network (4) from the intensity and wavelength of the light reflected by the Bragg gratings and the intensity and kind of the underground.

10. Device for generating training data for a neural network (4), comprising
at least one optical waveguide (1) having a plurality of fiber Bragg gratings (15),
at least one light source (2) designed to couple light (20) into the optical waveguide (1)
at least one spectrometer (3) designed to determine a spectrum (35) of the light reflected in the optical waveguide (1) by measuring the intensity (I) versus the wavelength (λ) of the reflected light,
an apparatus for deforming (6) the optical waveguide (1), which contains or consists of a robot arm 60), and
an apparatus (7) for detecting the shape of the optical waveguide (1), from the position of the robot arm (60), and
a memory (80) in which the shape of the optical waveguide (1) and the spectrum can be stored.

11. Device according to claim 10, **characterized in that** the apparatus (7) for detecting the shape of the optical waveguide (1) contains a camera (70), which produces a data stream representing an image of the optical waveguide (1).

## Revendications

1. Procédé pour définir la forme d'un guide d'ondes optique (1) comportant une pluralité de réseaux de Bragg à fibres (15), comprenant les étapes suivantes consistant à :
injecter de la lumière (20) d'une source de lumière (2) dans le guide d'ondes optique (1),
extraire la lumière réfléchie du guide d'ondes optique (1),
définir un spectre (35) de la lumière réfléchie en mesurant l'intensité (I) en fonction de la longueur d'onde (λ),
**caractérisé en ce que**
le spectre (35) est amené à un réseau neuronal auto-apprenant (4) qui a été appris au préalable avec des données d'entraînement qui se composent d'une pluralité de jeux de données qui contiennent chacun un spectre mesuré (35) et la forme associée du guide d'ondes optique (1), la forme du guide d'ondes optique (1) étant déterminée avec le réseau neuronal (4) à partir de l'intensité et de la longueur d'onde de la lumière réfléchie sur les réseaux de Bragg et de l'intensité et du type de sous-sol.

2. Procédé selon la revendication 1,
**caractérisé en ce que** le réseau neuronal auto-apprenant est appris avec des données d'entraînement qui se composent d'une pluralité de jeux de données qui contiennent chacun un spectre mesuré (35) et la forme associée du guide d'ondes optique (1).

3. Procédé selon la revendication 2,
**caractérisé en ce que** les données d'entraînement contiennent plus de 600 ou plus de 900 ou plus de 4 000 ou plus de 10 000 ou plus de 100 000 jeux de données.

4. Procédé selon l'une des revendications 2 ou 3,
**caractérisé en ce que** les données d'entraînement sont créées de manière automatisée avec un dispositif (5) conçu pour déformer le guide d'ondes optique (1), pour détecter la forme et pour mesurer le spectre (35) associé à la forme respective.

5. Procédé selon la revendication 4,
**caractérisé en ce que** la déformation du guide d'ondes optique (1) est réalisée par un bras de robot (60).

6. Procédé selon la revendication 5,
**caractérisé en ce que** la détection de la forme du guide d'ondes optique (1) est réalisée à partir de la position du bras de robot (60).

7. Procédé selon l'une des revendications 4 ou 6,
**caractérisé en ce que** la détection de la forme du guide d'ondes optique (1) est réalisée par traitement électronique d'image à partir d'au moins une prise de vue du guide d'ondes optique (1).

8. Utilisation d'un procédé selon l'une des revendications 1 à 7 pour définir la forme d'un cathéter ou d'un endoscope ou d'une aiguille de biopsie ou d'un profil aérodynamique.

9. Dispositif pour définir la forme d'un guide d'ondes optique (1), comprenant au moins un guide d'ondes optique (1) comportant une pluralité de réseaux de Bragg à fibres (15),
au moins une source de lumière (2) conçue pour injecter de la lumière dans le guide d'ondes optique (1),
au moins un spectromètre (3) conçu pour définir un spectre (35) de la lumière réfléchie dans le guide d'ondes optique (1) en mesurant l'intensité (I) en fonction de la longueur d'onde (λ) de la lumière réfléchie,
**caractérisé en ce que**
le dispositif comprend en outre un réseau neuronal auto-apprenant (4) qui a été appris au préalable avec des données d'entraînement qui se composent d'une pluralité de jeux de données qui contiennent chacun un spectre mesuré (35) et la forme associée du guide d'ondes optique (1), et qui présente au moins une entrée (41) et au moins une sortie (42), le spectre pouvant être amené à l'entrée (41), et des données représentant la courbure et/ou la torsion pouvant être fournies à la sortie (42), lesquelles peuvent être déterminées avec le réseau neuronal à partir de l'intensité et de la longueur d'onde de la lumière réfléchie sur les réseaux de Bragg et de l'intensité et du type de sous-sol.

10. Dispositif pour générer des données d'entraînement pour un réseau neuronal (4), comprenant
au moins un guide d'ondes optique (1) comportant une pluralité de réseaux de Bragg à fibres (15),
au moins une source de lumière (2) conçue pour injecter de la lumière (20) dans le guide d'ondes optique (1),
au moins un spectromètre (3) conçu pour définir un spectre (35) de la lumière réfléchie dans le guide d'ondes optique (1) en mesurant l'intensité (I) en fonction de la longueur d'onde (λ) de la lumière réfléchie,
un moyen (6) de déformation du guide d'ondes optique (1), qui comprend un bras de robot (60) ou en est constitué, et
un moyen (7) de détection de la forme du guide d'ondes optique (1) à partir de la position du bras de robot (60), et
une mémoire (80) dans laquelle la forme du guide d'ondes optique (1) et le spectre peuvent être stockés.

11. Dispositif selon la revendication 10,
**caractérisé en ce que** le moyen (7) de détection de la forme du guide d'ondes optique (1) comprend une caméra (70) qui génère un flux de données représentant une image du guide d'ondes optique (1).
